# EUROPÄISCHE PATENTSCHRIFT

(11) **EP 2 370 489 B1**
(45) Veröffentlichungstag und Bekanntmachung des Hinweises auf die Patenterteilung: **02.01.2013**
(21) Anmeldenummer: 09767940.1
(22) Anmeldetag: 11.11.2009
(51) Int. Cl.: C08G 18/28, C08G 18/32, C08G 18/10, C08G 18/80, C07C 273/18, C07C 275/40, C09D 5/04

(54) **VERFAHREN ZUR HERSTELLUNG RHEOLOGISCH WIRKSAMER HARNSTOFFURETHANE IN ORGANISCHEN SALZEN**
METHOD FOR PRODUCING RHEOLOGICALLY EFFECTIVE UREA URETHANES IN ORGANIC SALTS
PROCÉDÉ DE PRODUCTION D'URÉTHANES URÉES À ACTIVITÉ RHÉOLOGIQUE DANS DES SELS ORGANIQUES

(30) Priorität: 01.12.2008 DE 102008059702
(43) Veröffentlichungstag der Anmeldung: 05.10.2011
(73) Patentinhaber: BYK-Chemie GmbH, 46483 Wesel (DE)
(72) Erfinder: ORTH, Ulrich, 46485 Wesel (DE); EBERHARDT, Marc, 46485Wesel (DE); OMEIS, Jürgen, 46286 Dorsten-Lembeck (DE); DAMS, Christian, 46509 Xanten (DE)
(74) Vertreter: Leifert & Steffan
(86) Internationale Anmeldenummer: PCT/EP2009/008043
(87) Internationale Veröffentlichungsnummer: WO 2010/063358

(56) Entgegenhaltungen:
- EP-A1- 1 188 779
- US-A1- 2002 115 882

## Beschreibung

Die Erfindung betrifft ein Verfahren zur Herstellung einer als Thixotropie-Mittel wirksamen, Harnstoffurethane enthaltenden Lösung, bei dem Monohydroxyverbindungen mit einem Überschuß von Toluylendiisocyanat umgesetzt werden, der nicht umgesetzte Teil des Toluylendiisocyanats aus dem Reaktionsgemisch entfernt wird und die erhaltenen Monoisocyanat-Addukte mit Diaminen in einer ionischen Flüssigkeit zu Harnstoffurethanen weiter umgesetzt werden. Die Erfindung betrifft des Weiteren die Verwendung der Lösung zur Thixotropierung von Beschichtungsmitteln.

Um die Rheologie von flüssigen Beschichtungssystemen zu steuern, werden auch heute vorwiegend organisch modifizierte Bentonite, Kieselsäuren, hydriertes Rizinusöl und Polyamidwachse eingesetzt. Nachteilig bei diesen Stoffen ist, daß sie meist trockene Feststoffe darstellen, die mittels Lösungsmitteln und Scherkräften zu einem Halbfabrikat aufgeschlossen bzw. durch gezielte Temperatursteuerung in das flüssige Beschichtungssystem eingebracht werden müssen. Werden diese Temperaturen nicht eingehalten, treten im fertigen Beschichtungssystem Kristallite auf, die zu Fehlern in der Beschichtung führen können.

Der generelle Nachteil dieser rheologischen Hilfsstoffe ist, dass sie zu Trübungen und Schleierbildungen (Haze) in klaren, transparenten Beschichtungen führen. Außerdem ist der Umgang mit trockenen, pulverförmigen Produkten, die Stäube bei der Verarbeitung verursachen, nicht gewünscht.

Andere Lösungen zur Rheologiesteuerung wurden in der europäischen Patentanmeldung EP-A-0 198 519 dargestellt. Hier wird ein Isocyanat mit einem Amin in Gegenwart von Lackharz-Lösungen zu einem Harnstoff umgesetzt, der in feinstdisperser Form nadelförmige Kristalle bildet. Diese so modifizierten Lackbindemittel werden als rheologiesteuernde und ablaufverhindernde Bindemittel, als sogenannte "sag control agents", angeboten. Der Nachteil dieser Produkte liegt darin begründet, dass sie immer an die Bindemittel gebunden sind, in denen sie hergestellt wurden und keine nachträgliche universelle Korrektur von fertigen Beschichtungsmitteln zulassen.

Im europäischen Patent EP-B-0 006 252 wird ein Verfahren zur Herstellung eines Thixotropie-Mittels beschrieben, das einige der oben genannten Nachteile ausräumt, indem es Harnstoffurethane beschreibt, die in aprotischen Lösungsmitteln in Gegenwart von Lithiumsalzen durch Umsetzung von Isocyanataddukten mit Polyaminen hergestellt werden. Die so hergestellten Produkte weisen jedoch zwei signifikante Nachteile auf. Zum einen sind diese Thixotropie-Mittel durch eine durch das Herstellungsverfahren bedingte undefinierte Struktur gekennzeichnet. Diese Produkte neigen dann zu Ausfällungen und sind nur unter größten Schwierigkeiten in Lösung zu halten. Zum anderen liegt ein weiterer Nachteil der durch dieses Verfahren hergestellten Thixotropie-Mittel darin, daß nur Monoisocyanat-Addukte gleicher Struktur mit dem Diamin umgesetzt werden. Dies führt wiederum zu einer begrenzten Verträglichkeit in den eingesetzten Beschichtungssystemen, die sich in Gelkörpern oder starken Trübungen äußert, und des Weiteren zu einer schlechteren rheologischen Wirksamkeit.

Ausgehend vom zuvor genannten Stand der Technik stellte die EP-B-1 188 779 Thixotropiermittel zur Verfügung, welche eine definierte Struktur aufweisen, eine größere Lagerstabilität der Lösung über mehrere Monate zulassen, sich durch eine breitere Verträglichkeit in Bindemitteln auszeichnen und damit eine sichere Anwendung der Produkte ermöglichen. Die Herstellung dieser Thixotropiermittel erfolgte in aprotischem Medium, insbesondere N-Methylpyrrolidon (NMP), in Gegenwart größerer Mengen an Lithiumsalzen, insbesondere Lithiumchlorid.

Sowohl mit dem Einsatz von N-Methylpyrrolidon als auch mit dem Einsatz von Lithiumsalzen sind jedoch Nachteile verbunden. N-Methylpyrrolidon und andere in der europäischen Patentschrift EP-B-1 188 779 genannte Lösemittel sind toxikologisch bedenklich und daher für eine weitere Verwendung in Lacksystemen nicht mehr länger akzeptabel. Der zwingende Einsatz von Lithiumsalzen und anderen Metallsalzen wiederum besitzt den Nachteil, dass diese nur begrenzt in den organischen Lösemitteln löslich sind. Da derartige Salze in proportionalen Mengen zu den für die Harnstoffurethan-Synthese notwendigen Addukten eingesetzt werden, können keine hochkonzentrierten Produktlösungen erhalten werden, ohne daß dies mit einer unerwünschten Teilausfüllungen der Salze verbunden wäre. Aus produktions- und lagertechnischen Gründen sind jedoch höher konzentrierte Lösungen von Harnstoffurethanen vorteilhaft. Wird zudem Lithiumchlorid eingesetzt, so treten bei der Beschichtung korrosionsempfindlicher Substrate unter Verwendung Lithiumchlorid enthaltender Thixotropiermittel in den Beschichtungsmitteln häufig Substratschädigungen auf.

Aufgabe der vorliegenden Erfindung war es daher ausgehend von der europäischen Patentschrift EP-B-1188779, die in dieser erzielten Vorteile der Zurverfügungstellung von Thixotropiermitteln, welche eine definierte Struktur aufweisen, eine gute Lagerstabilität der Lösung über mehrere Monate besitzen, sich durch eine breite Verträglichkeit in Bindemitteln auszeichnen und damit eine sichere Anwendung ermöglichen beizubehalten und die Nachteile des Einsatzes von Lithiumsalzen sowie N-Methylpyrrolidon oder vergleichbar schädlichen Lösemitteln bei deren Herstellung und Lagerung zu vermeiden.

Überraschenderweise wurde gefunden, dass diese Aufgaben dadurch gelöst werden konnten, dass bei dem in der europäischen Patentschrift EP-B-1188779 offenbarten Verfahren sowohl die dort angegebenen aprotischen Lösemittel als auch die eingesetzten Lithiumsalze durch ionische Flüssigkeiten ersetzt werden.

Ionische Flüssigkeiten sind auf Grund ihrer relativ hohen Molekulargewichte sowie ihres ionogenen Charakters in der Regel schwer bis gar nicht flüchtig und können daher als frei von flüchtigen organischen Verbindungen angesehen werden. Da es in ionischen Flüssigkeiten sehr viele Kombinationsmöglichkeiten von Kationen und Anionen gibt, kann durch geeignete Wahl des die ionische Flüssigkeit bildenden Kations und Anions die Verträglichkeit und damit Lagerstabilität für unterschiedliche Bindemittelsysteme deutlich besser angepasst werden, als es mit dem Lösemittelsystem aus NMP und Metallsalz möglich ist, da die Metallsalze nur sehr begrenzt in organischen Lösemitteln löslich sind. Ein weiterer überraschender Vorteil ist, dass mit ionischen Flüssigkeiten der Wirkstoffanteil der Thixotropiermittel bei gleichzeitig sehr guter Lagerstabilität deutlich erhöht werden kann.

Die vorliegende Erfindung betrifft demnach ein Verfahren, bei dem mindestens zwei strukturell verschiedene Monohydroxyverbindungen der allgemeinen Struktur R-OH, in der R für einen n-Alkyl-Rest oder einen iso-Alkyl-Rest mit 4 bis 22 Kohlenstoffatomen, einen Alkenylrest mit 3 bis 18 Kohlenstoffatomen, einen Cycloalkylrest mit 3 bis 8 Kohlenstoffatomen, einen Aralkylrest mit 5 bis 12 Kohlenstoffatomen, oder für einen Rest der Formel CₘH₂ₘ₊₁(O-CₙH₂ₙ)ₓ-, CₘH₂ₘ₊₁(OOC-CᵥH₂ᵥ)ₓ- oder Z-C₆H₄(O-CₙH₂ₙ)ₓ-, in denen m=1-22 ist, n=2-4 ist, x=1-15 ist, v=4 oder 5 ist und Z einem Alkylrest mit 1 bis 12 Kohlenstoffatomen entspricht, steht, mit einem 1,5 bis 5-fachen Überschuß von Toluylendiisocyanat zu Monoisocyanataddukten umgesetzt werden, und der nicht umgesetzte Teil des Toluylendiisocyanats aus dem Reaktionsgemisch entfernt wird,
die auf diese Weise erhaltenen Monoisocyanat-Addukte mit Diaminen der Formel H₂N-R'-NH₂, in der R' einem Rest
CₒH₂ₒ mit o = 2 bis 12,
(CₚH₂ₚ-O)_{q}-CₚH₂ₚ mit p = 2 bis 4 und q = 1 bis 25, 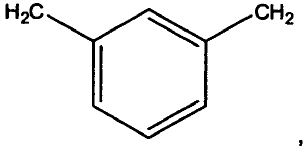 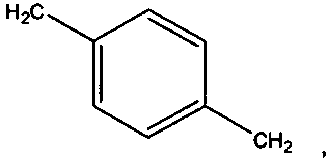 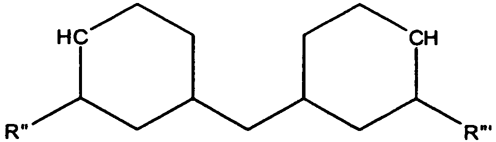 (R" und R'" stehen unabhängig voneinander für CH₃ oder H), oder 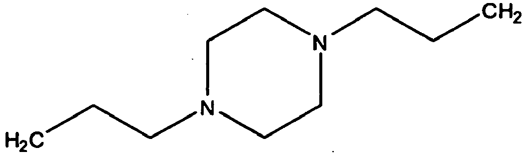 entspricht, oder Mischungen davon, in einer ionischen Flüssigkeit zu Harnstoffaddukten umsetzt, die zwei unterschiedliche Reste R tragen.

Das Verfahren bedarf nicht des Einsatzes lösungsstabilisierender Lithiumverbindungen, weshalb diese vorzugsweise im Thixotropiermittel, das heißt der Harnstoffurethane enthaltenden Lösung, nicht enthalten sind. Des Weiteren werden bei der Umsetzung der Monoisocyanat-Addukte mit Diaminen neben den ionischen Flüssigkeiten vorzugsweise keine weiteren Lösemittel, insbesondere keine toxikologisch bedenklichen Lösemittel eingesetzt. Werden weitere von den ionischen Flüssigkeiten unterschiedliche Lösemittel, insbesondere toxikologisch unbedenkliche Lösemittel eingesetzt, so liegt deren Gehalt vorzugsweise unter 20 Gew.-% bezogen auf das Gesamtgewicht aller Lösemittel. Werden derartige Lösemittel eingesetzt, so sind diese vorzugsweise aprotisch. Des Weiteren enthalten die nach dem erfindungsgemäßen Verfahren erhältlichen Thixotropiermittel vorzugsweise keine Halogenide, insbesondere keine Chloride.

Die als Thixotropier-Mittel wirksame, Harnstoffurethane enthaltende Lösung kann durch das erfindungsgemäße Verfahren grundsätzlich auf zwei verschiedenen Wegen erhalten werden:
a) zum einen können zunächst mindestens zwei strukturell verschiedene Alkohole R-OH vermischt und anschließend mit einem 1,5 bis 5-fachen Überschuss von Toluylendiisocyanat umgesetzt werden. Der nicht umgesetzte Teil des Totuyiendiisocyanats wird danach unter schonenden Bedingungen, dem Stand der Technik gemäß, aus dem Reaktionsgemisch entfernt, und die auf diese Weise erhaltene Mischung aus den strukturell verschiedenen Monoisocyanat-Addukten wird anschließend mit den Diaminen in einer ionischen Flüssigkeit zu Harnstoffurethanen umgesetzt.
b) zum anderen können zunächst mindestens zwei strukturell verschiedene Alkohole R-OH getrennt voneinander mit einem 1,5 bis 5-fachen Überschuss von Toluylendiisocyanat umgesetzt werden. Der nicht umgesetzte Teil des Toluylendiisocyanats wird unter schonenden Bedingungen, dem Stand der Technik gemäß, aus dem jeweiligen Reaktionsgemisch entfernt, und die auf diese Weise erhaltenen, strukturell verschiedenen Monoisocyanat-Addukte werden miteinander vermischt. Die so erhaltene Mischung aus strukturell verschiedenen Monoisocyanat-Addukten wird anschließend mit den Diaminen in einer ionischen Flüssigkeit zu Harnstoffurethanen der allgemeinen Struktur (II) umgesetzt.

Der molare Anteil der jeweiligen Monoisocyanat-Addukte in der Mischung aus strukturell verschiedenen Monoisocyanat-Addukten liegt zwischen 20 und 80 %, bevorzugt zwischen 35 und 65 %, besonders bevorzugt zwischen 45 und 55 %, wobei die Summe der molaren Anteile der Monoisocyanat-Addukte 100 % beträgt.

Der molare Überschuss des Toluylendiisocyanats beträgt bevorzugt 1,5 bis 5,0 Mol, besonders bevorzugt 2,0 bis 4,0 Mol.

Der Feststoffgehalt der so erzeugten Harnstoffurethan-Lösungen beträgt 5 bis 80 Gew.-%, bevorzugt 20 bis 60 Gew.-%, besonders bevorzugt 25 bis 50 Gew.-%, bezogen auf das Gesamtgewicht der erzeugten Harnstoffurethan-Lösung. Unter "Feststoffgehalt" wird hierin der rechnerische Gehalt an Harnstoffurethan verstanden, welcher sich aus der Summe der Gewichtsprozente des Amins und des Isocyanatmonoadduktes ergibt.

Bei den zur Herstellung der Monoisocyanat-Addukte eingesetzten Alkoholen R-OH handelt es sich bevorzugt um lineare oder verzweigte primäre Alkohole, die gesättigt oder ungesättigt sein können wie z.B. n-Butanol, 2-Ethylhexanol, Isotridecylalkohol, Guerbet-Alkohole der Kettenlänge C₁₀ bis C₂₀, Oleylalkohol, Linoleylalkohol, Laurylalkohol, Stearylalkohol, aber auch cycloaliphatische Alkohole wie z.B. Cyclohexanol oder dessen alkylsubstituierte Derivate sowie aromatisch substituierte Alkanole wie Benzylalkohol sind geeignet.

Zur Einstellung der Polarität sind besonders die alkoxylierten Derivate der oben aufgeführten Alkohole geeignet, wobei in diesem Falle auch niedere Alkohole mit 1 bis 6 Kohlenstoffatomen wie z.B. Methanol oder Allylalkohol als Startkomponente für die Alkoxylierung verwendet werden können. Die so hergestellten Produkte enthalten unter anderem bevorzugt Ethylenoxid- und/oder Propylenoxid- und/oder Butylenoxid- und/oder Styroloxideinheiten in der Kette und können diese Einheiten alternierend oder in Blöcken aufweisen. Besonders bevorzugt sind Ethylenoxid- und/oder Propylenoxideinheiten. Bei der Alkoxylierung können auch aromatische Alkohole wie beispielsweise Phenole oder Alkylphenole als Startkomponente eingesetzt werden.

Um die Verträglichkeit der erfindungsgemäßen Harnstoffurethane an moderne Bindemittel anzupassen, ist es auch möglich, Ester- oder Polyestergruppen in die Alkoholkomponente einzubringen, z.B. durch Addition von Lactonen, wie z.B. ε-Caprolacton, an die oben aufgeführten Alkohole oder Alkoholalkoxylate, oder durch die Verwendung hydroxyfunktioneller (Meth)acrylate.

Bei den Diisocyanaten, welche zur Bildung der Monoisocyanat-Addukte eingesetzt werden, handelt es sich im wesentlichen um Toluylendiisocyanate in der bekannten und üblichen Isomerenverteilung wobei sich bei der Destillation der Überschussanteile an Diisocyanat Verschiebungen im Isomerenanteil ergeben, so daß auch höhere Anteile an 2,6-Toluylendisocyanat entstehen können als üblicherweise im Handel angeboten wird. Diese Destillate können bei der Herstellung weiterer Monoaddukte wieder eingesetzt werden. Bevorzugt sind Toluylendiisocyanat-Isomere mit 50 bis 100 Mol-% 2,4-Isomerenanteil.

Bei den Diaminen der Formel H₂N-R'-NH₂ handelt es sich im wesentlichen um lineare Diamine mit der Kohlenstoffkettenlänge von 2 bis 12 Kohlenstoffatomen, die geradkettig oder verzweigt sein können, wie z.B. 1,3-Propandiamin, Hexamethylendiamin, Octamethylendiamin, Diaminododecan oder Neopentandiamin. Cyclische Diamine wie z.B. 4,4'-Diaminodicyclohexylmethan oder 3,3'-Dimethyl-4,4'-diaminodicyclohexylmethan sind ebenfalls geeignet. Besonders bevorzugt sind aromatisch-aliphatische Diamine wie z.B. meta- oder para-Xylylendiamin. Die Diamine können auch als Mischung zur Harnstoffbildung eingesetzt werden, da hierdurch die Kristallisationsneigung des Harnstoffurethans in Lösung reduziert wird. Die durch das erfindungsgemäße Verfahren hergestellten Harnstoffurethane enthalten weder freie Isocyanat- noch freie Aminogruppen. "Frei von Isocyanat- und Aminogruppen" bedeutet hierbei, daß der Restgehalt an Isocyanatgruppen kleiner 0,01 % bezogen auf die eingesetzte Menge des Monoadduktes ist und der Gehalt an primären Aminogruppen kleiner 0,05% bezogen auf die Menge des eingesetzten Diamins ist. Der Gehalt an NCO- bzw. NH₂-Gruppen lässt sich hierbei titrimetrisch bestimmen. Sie sind dementsprechend physiologisch unbedenklich. Des Weiteren treten keine negativen Nebenreaktionen mit Bindemitteln oder Füllstoffen auf. Die Lagerstabilität dieser so hergestellten Harnstoffurethan-Lösungen ist außerordentlich hoch und beträgt bei normaler Lagertemperatur durchaus 12 Monate oder mehr. Die Harnstoffurethan-Lösungen weisen zudem eine breite Verträglichkeit in Bindemitteln auf und ermöglicht damit eine sichere Anwendung der Thixotropie-Mittel.

Die Umsetzung der Monoisocyanat-Adduktmischungen mit dem Diamin erfolgt in einer ionischen Flüssigkeit. Unter "ionischen Flüssigkeiten" werden im Rahmen der vorliegenden Erfindung organische Salze oder Gemische aus organischen Salzen verstanden, deren Schmelzpunkte unterhalb 80°C liegen, vorzugsweise liegen deren Schmelzpunkte unter 50°C, besonders bevorzugt unter 30°C und ganz besonders bevorzugt unter 20°C. Die hierin besonders bevorzugten ionischen Flüssigkeiten sind bei Raumtemperatur (25°C) flüssig.

Mehrere Veröffentlichungen beschreiben bereits die Verwendung ionischer Flüssigkeiten als Lösungsmittel für Übergangsmetall-katalysierte Reaktionen (Übersichtsartikel: T. Welton, Chem. Rev. 1999, 99, 2071) Beispielsweise wurden Hydrierungen von Olefinen mit Rhodium(I)) (P. A. Z. Suarez, J. E. L. Dullius, S. Einloft, R. F. de Souza und J. Dupont, Polyhedron 15/7, 1996, 1217-1219), Ruthenium(II) und Cobalt(II) komplexen (P. A. Z. Suarez, J. E. L. Dullius, S. Einloft, R. F. de Souza und J. Dupont, Inorganica Chimica Acta 255, 1997, 207-209) in ionischen Flüssigkeiten mit Tetrafluoroborat-Anion erfolgreich bearbeitet. Auch die Hydroformylierung von funktionalisierten und unfunktionalisierten Olefinen gelingt mit Rhodium-Katalysatoren in ionischen Flüssigkeiten mit schwach koordinierenden Anionen (z.B. PF₆⁻, BF4) EP-A-0776880, Y. Chauvin, L. Mussmann, H. Olivier, Angew. Chem., Int. Ed. Engl., 1995, 34, 2698; W. Keim, D. Vogt, H. Waffenschmidt, P. Wasserscheid, J. of Cat., 1999, 186, 481).

Zur Synthese von binären ionischen Flüssigkeiten vom Typ [A]⁺[Y]⁻ kann beispielsweise ein zweistufiges Verfahren angewendet (J. S. Wilkes, M. J. Zaworotko, J. Chem. Soc., Chem. Commun., 13, 1992, 965) werden. Dabei wird zunächst durch Reaktion eines Alkylierungsreagenz RX und eines Amins NR¹R²R³ oder eines Phosphans PR¹R²R³ in einer Quarternisierungsreaktion das organische Ammoniumsalz [NR¹R²R³R]⁺ X⁻ oder das organische Phosphoniumsalz [PR¹R²R³R]⁺ X⁻ aufgebaut. X⁻ ist dabei in der Regel ein Halogenid-Ion. Das organische Halogenidsalz wird isoliert und in einer nachfolgenden, zweiten Reaktionsstufe in einer Austauschreaktion mit dem Alkali- oder Erdalkalisalz des Typs M⁺ [Y]⁻ umgesetzt. Dies geschieht in einem Lösungsmittel, in dem das Nebenprodukt M⁺ X⁻ schwerlöslich, die zu synthetisierende ionische Flüssigkeit [A]⁺[Y]⁻ dagegen gut löslich ist. Sollen ionische Flüssigkeiten erhalten werden, die als Anion ein Halogenid-Anion besitzen, so kann die Synthese nach der ersten Stufe abgebrochen werden.

Das zweistufige Verfahren wurde in der Literatur beispielsweise zur Darstellung von ionischen Flüssigkeiten mit [BF₄]⁻, [PF₆]⁻-, Acetat-, Nitrat-, HSO₄⁻-, SO₄²⁻-Ionen erfolgreich verwendet (J. S. Wilkes, M. J. Zaorotko, J. Chem. Soc., Chem. Commun., 13, 1992, 965, B. Ellis, *WO 9618459 A1* 960620, **1996** J. Fuller, R. T. Carlin, H. C. de Long, D. Haworth, J. Chem. Soc., Chem. Commun., 3, 1994, 299).

Von Nachteil bei dieser Reaktionsführung ist es, dass nur dann ein quantitativer Austausch des Halogenidsalzes [NR¹R²R³R]⁺X⁻ bzw. [PR¹R²R³R]⁺X⁻ zur gewünschten ionischen Flüssigkeit [NR¹R²R³R]⁺[Y]⁻ bzw. [PR¹R²R³R]⁺[Y]⁻ gelingt, wenn unter Austauschbedingungen das Reaktionssystem vollständig wasserfrei ist. Dies ist insbesondere dann von Bedeutung, wenn möglichst halogenidfreie ionische Flüssigkeiten erhalten werden sollen.

Da Halogenid-Ionen für zahlreiche Anwendungen der erfindungsgemäßen Thixotropiermittel nachteilig sind - sie wirken bei einigen Anwendungen korrosionsfördernd und in anderen Anwendungen als Katalysatorgift - ist es von Vorteil insbesondere solche ionischen Flüssigkeiten zur Herstellung der erfindungsgemäßen Thixotropiermittel zu verwenden, die kein Halogenid-Anion enthalten. Unter "halogenidfreien" ionischen Flüssigkeiten werden solche verstanden, die als Anion keine nennenswerten Mengen Halogenid-Ionen enthalten, vorzugsweise ist der Gehalt an Halogenid-Ionen kleiner 0,1 Gew.-%, besonders bevorzugt kleiner 0,05 Gew.-% und ganz besonders bevorzugt kleiner 0,01 Gew.-% bezogen auf das Gesamtgewicht der ionischen Flüssigkeit.

Ein einstufiges Verfahren zur Herstellung derartiger praktisch (nahezu) halogenidfreier ionischer Flüssigkeiten offenbart die EP-A-1 182 197. Die nach diesem Verfahren erhältlichen Produkte können auch im Verfahren der vorliegenden Erfindung als Reaktionsmedium bevorzugt eingesetzt werden.

Besonders bevorzugte im erfindungsgemäßen Verfahren verwendbare ionische Flüssigkeiten besitzen die allgemeine Formel (I)

[A]ₙ⁺[Y]ⁿ⁻,

wobei n = 1 oder 2 ist und
das Anion [Y]ⁿ⁻ gewählt ist aus der Gruppe bestehend aus Tetrafluoroborat [BF₄]⁻, Tetrachloroborat [BCl₄]⁻, Phosphat [PO₄]³⁻, Alkylphosphat [ROPO₃]²⁻ / [ROR'OPO₂]⁻, Hexafluorophosphat [PF₆]⁻, Hexafluoroantimonat [SbF₆]⁻, Hexafluoroarsenat [AsF₆]⁻, Tetrachloroaluminat [AlCl₄]⁻, Trichlorozinkat [ZnCl₃]⁻, Dichlorocuprat [CuCl₂]⁻, Sulfat [SO₄]²⁻, Alkylsulfat [R'-SO₄]-, Carbonat [CO₃]²⁻, Fluorosulfonat, [R'-COO]⁻, [R'-SO₃]⁻ oder [(R'-SO₂)₂N]⁻, und R und R' unabhängig voneinander ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-ArylRest ist, dessen Wasserstoffatome ganz oder teilweise durch Halogenatome substituiert sein können. Wie bereits erwähnt kann das Anion [Y]ⁿ⁻ auch für ein Halogenid wie [F]⁻, [Cl]⁻, [Br]⁻ oder [I]⁻ stehen, womit jedoch je nach Einsatzzweck der Thixotropiermittel die oben beschriebenen Nachteile (Korrosion) verbunden sein können.

Das Kation [A]⁺ ist vorzugsweise gewählt aus quarternären Ammonium-Kationen, Phosphonium-Kationen oder Kationen von N-haltigen Heterozyklen. Besonders bevorzugte Kationen lassen sich durch die nachfolgenden Strukturen wiedergeben: 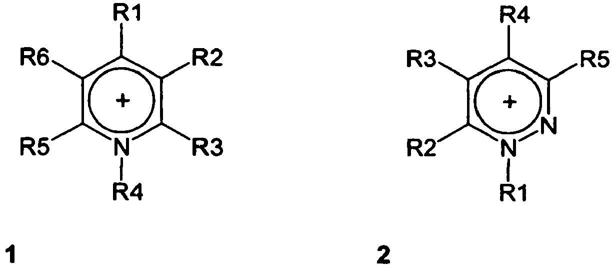 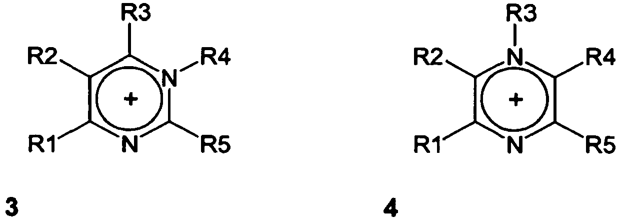 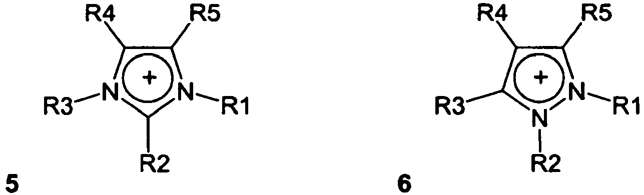 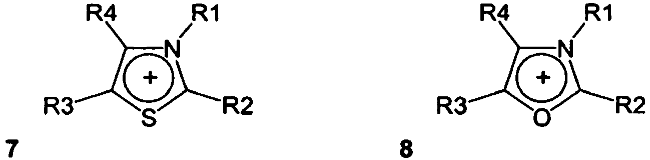 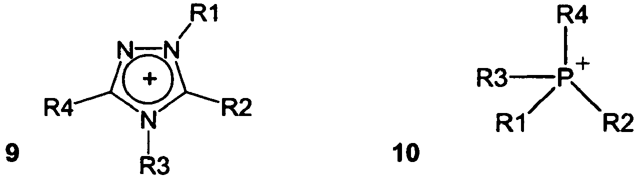 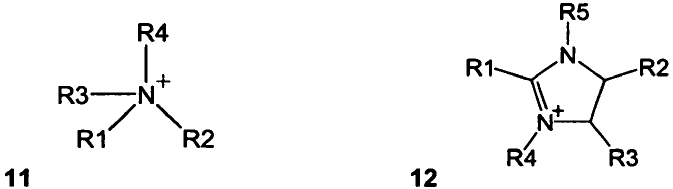 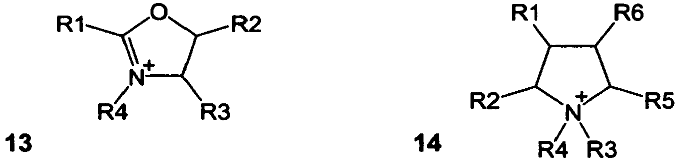 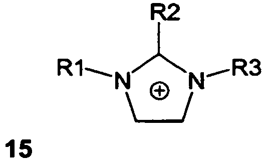 wobei die Reste R1, R2, R3, R4, R5 und R6 unabhängig voneinander gewählt sind aus der Gruppe bestehend aus (i) linearen oder verzweigten, gesättigten oder ungesättigten, aliphatischen oder alicyclischen Alkylgruppen mit 1 bis 20 Kohlenstoffatomen, die substituiert, beispielsweise mit einer Alkylgruppe mit 1-8 Kohlenstoffatomen und/oder Halogengruppe, oder unsubstituiert sein können; (ii) Heteroaryl-, Heteroaryl-C₁-C₆-Alkylgruppen mit 3 bis 8 Kohlenstoffatomen im Heteroarylrest und wenigstens einem Heteroatom ausgewählt aus N, O und S, der mit wenigstens einer Gruppe ausgewählt aus C₁-C₆-Alkylgruppen und/oder Halogenatomen substituiert sein können; (iii) Aryl-, Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenatom substituiert sein können; (iv) ein durch ein oder mehrere Heteroatome (N, O) unterbrochener linearer oder verzweigter aliphatischer und/oder cycloaliphatischer und/oder aromatischer Kohlenwasserstoffrest mit 2 - 40 Kohlenstoffatomen, der substituiert, beispielsweise mit einer Alkylgruppe mit 1-8 Kohlenstoffatomen und/oder Halogengruppe, oder unsubstituiert sein kann; (v) ein durch ein oder mehrere Funktionalitäten, ausgewählt aus der Gruppe -O-C(O)-, -(O)C-O-, -NH-C(O)-, -(O)C-NH-, -(CH₃)N-C(O)-, -(O)C-N(CH₃)-, -S(O)₂-O-, -O-S(O)₂-, -S(O)₂-NH-, -NH-S(O)₂-, -S(O)₂-N(CH₃)-, -N(CH₃)-S(O)₂-, unterbrochener linearer oder verzweigter aliphatischer Kohlenwasserstoffrest mit 2 - 20 Kohlenstoffatomen, der substituiert, beispielsweise mit einer Alkylgruppe mit 1-8 Kohlenstoffatomen und/oder Halogengruppe, oder unsubstituiert sein kann. Sind Wasserstoffatome an den oben genannten Resten R1, R2, R3, R4, R5 oder R6 substituiert, so tragen die Substituenten keine gegenüber Isocyanatgruppen reaktiven Gruppen wie beispielsweise Hydroxy- oder Aminogruppen.

In einer besonderen Ausgestaltung der Erfindung können die Wasserstoffatome an Alkyl-, Aryl-, Arylalkyl- und Alkylaryl-Sulfonatgruppen durch Halogenatome, insbesondere Fluor, Chlor oder Brom substituiert sein. Besonders bevorzugt sind die fluorierten, insbesondere die perfluorierten Alkyl- und oben genannten Arylsulfonate, wie das Trifluormethansulfonat (Triflat). Als nicht halogenierte Vertreter sind die Methansulfonat-, Benzolsulfonat- und die Toluolsulfonat-Gruppe zu nennen, sowie alle weiteren im Stand der Technik bekannten Sulfonat-Austrittsgruppen.

In einer weiteren Ausgestaltung der Erfindung können die Wasserstoffatome an Alkyl-, Aryl-, Arylalkyl- und Alkylaryl-Carboxylatgruppen durch Halogenatome, insbesondere Fluor, Chlor oder Brom substituiert sein. Besonders bevorzugt sind die fluorierten, insbesondere die perfluorierten Alkyl- und obengenannten Arylcarboxylate, wie das Trifluormethancarboxylat (Trifluoracetat; CF₃COO⁻). Als nicht halogenierte Vertreter sind die Acetat- und Benzoat-Gruppe zu nennen, sowie alle weiteren im Stand der Technik bekannten Carboxylat-Austrittsgruppen.

Das im Alkylierungsagens RX enthaltende Halogenidanion X⁻ ist üblicherweise ausgewählt aus der Gruppe bestehend aus Chlorid, Bromid und Iodid. Unter dem Begriff "Alkylierung" ist die Alkylierung selbst aber auch die Arylalkylierung und die Heteroarylalkylierung mit den angegebenen Gruppen zu verstehen.

Die Kationen der verwendeten ionischen Flüssigkeiten basieren bevorzugt auf Ammonium-, Pyridinium, Pyrrolidinium-, Pyrrolium-, Oxazolium-, Oxazolinium-, Imidazolium-, Thiazolium- oder Phosphoniumionen sowie Mischungen dieser Kationen. Die Kationen sollen sich chemisch inert gegenüber Isocyanatgruppen verhalten.

Besonders bevorzugt werden Kationen auf der Basis von Imidazolium- und Oxazoliumionen.

Die Anionen sind vorzugsweise ausgewählt aus Alkylsulfaten, Arylsulfaten, Sulfat, Hydrogensulfat, Phosphat, Alkylphosphaten, Arylphosphaten, Tosylaten, Alkylboraten, Halogenboraten wie z.B. Tetrafluoroborat, Halogenaluminaten wie z.B. Tetrachloroaluminat, Carboxylaten wie z.B. Acetat und Trifluoracetat, Perchlorat sowie Mischungen dieser Anionen.

Besonders bevorzugt sind Alkylsulfate, Tosylate und Acetate.

Die vorliegende Erfindung betrifft des Weiteren die Verwendung der durch das erfindungsgemäße Verfahren hergestellten Harnstoffurethan-Lösung zur Thixotropierung von Beschichtungsmitteln. Bei den Beschichtungsmitteln handelt es sich bevorzugt um wässrige, lösungsmittelhaltige und lösungsmittelfreie Lacke, PVC-Plastisole, Beschichtungen auf Epoxidbasis und auf Basis ungesättigter Polyesterharze.

Des Weiteren betrifft die Erfindung Lösungen der oben definierten asymetrischen Harnstoffurethane in ionischen Flüssigkeiten.

Die wesentlichen Merkmale des erfindungsgemäßen Verfahrens werden anhand der folgenden Ausführungsbeispiele verdeutlicht.

### BEISPIELE

In Tabelle 1 sind die im Folgenden verwendeten Monoaddukte aus TDI und einem Monoalkohol aufgeführt. Die Herstellung ist in der Patenschrift EP-B-1 188 779 ausführlich beschrieben. Beispiele 1 bis 3 der vorliegenden Erfindung entsprechen Beispielen 1 bis 3 der EP 1 188 779 B1 und Beispiele 4 bis 6 der vorliegenden Erfindung entsprechen Beispielen 5 bis 7 der EP 1 188 779 B1.

**Tabelle 1: Verwendte Monoaddukte 1 (gemäß Patentschrift EP-B-1 188 779):**

| **Beispiel** | **Alkohol** | **NCO-Gehalt** | **Equivalent-Gewicht** | **Mol-Verhältnis TDI : Alkohol** |
|---|---|---|---|---|
| 1 | Butanol | 16,9% | 248 | 2,5 : 1 |
| 2 | Butyltriglykol | 10,9% | 392 | 2,5 : 1 |
| 3 | iso-Tridecanol | 11,3% | 372 | 3 : 1 |
| 4 | MPEG 350 | 8,0 % | 525 | 3 : 1 |
| 5 | MPEG 500 | 6,2% | 675 | 3 : 1 |
| 6 | MPEG350 / MPEG500 (molares Verhältnis 1:1) | 7,0% | 600 | 2 : 1 |

**Tabelle 2: Mischungen der Monoaddukte aus Tabelle 1**

| **Mischung** | **Monoadduktmischung** | **Molares Verhältnis** |
|---|---|---|
| A | Butyltriglykol / iso-Tridecanol | 1 : 1 |
| | (aus Beispiel. 2 + 3) | |
| B | Butyltriglykol / MPEG 500 | 1 : 1,75 |
| | (aus Beispiel. 2+5) | |
| C | Butyltriglykol / MPEG 350 / MPEG 500 | 1 : 1 : 1 |
| | (aus Beispiel. 2 + 6) | |

### Vergleichsbeispiel (gemäß Patentschrift EP-B-1 188 779, Bsp. 8):

In 403 g N-Methylpyrrolidon werden bei 80°C 15,9 g LiCl und 68 g m-Xylylendiamin gelöst. Dann werden innerhalb von 1 Stunde 320 g der Mischung A zudosiert. Nach erfolgter Zugabe wird noch 30 Minuten gerührt und dann auf Raumtemperatur abgekühlt. Die so erhaltene Harnstoffurethanlösung hat einen Feststoffgehalt von 50%.

### Beispiel 1 (erfindungsgemäß):

Es wird eine Mischung aus 68 g m-Xylylendiamin und 690 g 1-Ethyl-3-methylimidazoliumethylsulfat hergestellt und auf 60°C erwärmt. Danach werden innerhalb von 1 Stunde 472 g der Mischung A zudosiert. Nach erfolgter Zugabe wird noch 30 Minuten gerührt und dann auf Raumtemperatur abgekühlt. Die so erhaltene Harnstoffurethanlösung hat einen Feststoffgehalt von 50%. Es wird ein klares, homogenes und flüssiges Produkt erhalten.

### Beispiel 2 (erfindungsgemäß):

Es wird eine Mischung aus 68 g m-Xylylendiamin und 238 g 1-Ethyl-3-methylimidazoliumethylsulfat hergestellt und auf 60°C erwärmt. Danach werden innerhalb von 1 Stunde 472 g der Mischung A zudosiert. Nach erfolgter Zugabe wird noch 30 Minuten gerührt und dann auf Raumtemperatur abgekühlt. Die so erhaltene Harnstoffurethanlösung hat einen Feststoffgehalt von 70%. Es wird ein klares, homogenes und flüssiges Produkt erhalten.

### Lagerstabilität

Es wird das Aussehen der Probe in Abhängigkeit der Zeit visuell beurteilt. Die Ergebnisse sind in Tabelle 3 zusammengefasst.

**Tabelle 3:**

| **Probe** | **Aussehen direkt nach Herstellung** | **Aussehen nach 1 Jahr** | **Gehalt Harnstoffurethan** |
|---|---|---|---|
| Vergleichs - beispiel | Stark trüb, inhomogen, Bildung von Bodensatz | Inhomogen, Bodensatz | 48% |
| Beispiel 1 | Klar, flüssig, homogen | Klar, flüssig, homogen | 50% |
| Beispiel 2 | Klar, flüssig, homogen | Klar, flüssig, homogen | 70% |

### Bestimmung des VOC-Gehaltes (Gehalt an flüchtigen organischen Verbindungen)

5 mg einer Probe der unter Beispiel der hergestellten Harnstoffurethanlösungen werden in ein 20ml Head-Space-Vial gefüllt und Luftdicht verschlossen. Dieses Vial, ein 7cm hohes Glasröhrchen mit 2 cm Durchmesser, wird 1 Stunde bei 100°C equilibriert und dann gaschromatographisch untersucht. Alle Peaks, die bei einer Retentionszeit zwischen C₆ und C₁₆ eluieren gehen in die Auswertung ein.
Folgendes Ergebnis wird erhalten:

**Tabelle 4:**

| **Probe** | **VOC-Gehalt** |
|---|---|
| Vergleichsbeispiel | 156.000 ppm |
| Beispiel 1 | < 150 ppm |
| Beispiel 2 | < 150 ppm |

Die Bestimmung wird durchgeführt mit einem Headspace-Sampler 7694 der Firma Agilent.

### Anwendungstechnische Ergebnisse

Zur Überprüfung der Antiabsetzwirkung der erfindungsgemäßen Harnstoffurethanlösungen wurden Pigmentslurries hergestellt und das Absetzverhalten nach Lagerung über einen Zeitraum von 3 Wochen überprüft.
Zur Herstellung der Pigmentslurries wird zunächst eine Mischung aus Wasser, Butylglykol, und Disperbyk 192 hergestellt. Diese Mischung wird dann unter Rühren dem Pigment, Iriodin 9303 Royal Gold WR II der Firma Merck, zugegeben. Anschließend werden ebenfalls unter Rühren mit dem Dispermaten, 2 Minuten bei einer Schergeschwindigkeit von 2 m/s, die erfindungsgemäßen Harnstoffurethanlösungen eingearbeitet.
Zur Beurteilung des Absetzverhaltens werden die Slurries in ein Glasgefäß (10cm hoch, 1,5cm Durchmesser) auf 7,5 cm Füllhöhe eingefüllt. Nach 3 Wochen Lagerung bei Raumtemperatur wird die Synerese bestimmt.

In Tabelle 5 sind die relativen Anteile der einzelnen Komponenten in der Mischung angegeben.

**Tabelle 5:**

| | **Control** | **Vergleichsbeispiel** | **Beispiel 1** |
|---|---|---|---|
| Iriodin 9303 Royal Gold WR II | 30,0 | 30,0 | 30,0 |
| Wasser | 64,5 | 64,5 | 64,5 |
| Butylglykol | 4,0 | 4,0 | 4,0 |
| Disperbyk 192 | 1,5 | 1,5 | 1,5 |
| Harnstoffurethanlösung | - | 1,0 | 1,0 |

**Tabelle 6: Antiabsetzwirkung**

| | **Control** | **Vergleichsbeispiel** | **Beispiel 1** |
|---|---|---|---|
| Gesamthöhe | 7,5 cm | 7,5 cm | 7,5 cm |
| Synerese | 4,2 cm | 0,2 cm | 0 cm |

### Korrosive Wirkung

Um die korrosive Wirkung der verwendeten Additive zu beurteilen wurden die in Tabelle 5 aufgeführten Lacke in Weißblechdosen gefüllt und verschlossen. Nach 6 Monaten wurde die Dose geöffnet und das Aussehen der inneren Dosenwand visuell beurteilt. Die Ergebnisse sind in Tabelle 7 aufgelistet.

**Tabelle 7: Korrosive Wirkung**

| | **Vergleichsbeispiel** | **Beispiel 1** |
|---|---|---|
| Aussehen der Dosenwand nach 6 Monaten | Dose ist an vielen Stellen teilweise stark gerostet | unverändert |

## Patentansprüche

1. Verfahren zur Herstellung einer als Thixotropie-Mittel wirksamen, Harnstoffurethane enthaltenden Lösung, bei dem Monohydroxyverbindungen der allgemeinen Struktur R-OH, in der R für einen n-Alkyl-Rest oder einen i-Alkyl-Rest mit 4 bis 22 C-Atomen, einen Alkenylrest mit 3 bis 18 C-Atomen, einen Cycloalkylrest oder einen Aralkylrest, ein Rest der Formel CₘH₂ₘ₊₁(O-CₙH₂ₙ)ₓ-, CₘH₂ₘ₊₁(OOC-CᵥH₂ᵥ)ₓ- oder Z-C₆H₄(O-CₙH₂ₙ)ₓ- mit m=1-22, n=2-4, x=1-15 und v=4 oder 5 und Z für einen Alkylrest mit 1 bis 12 C-Atomen steht, mit einem 1,5 bis 5-fachen Überschuß von Toluylendiisocyanat umgesetzt wird, der nicht umgesetzte Teil des Toluylendiisocyanats aus dem Reaktionsgemisch entfernt wird und die auf diese Weise erhaltenen Monoisocyanat-Addukte mit Diaminen der Formel H₂N-R'-NH₂, in der R'
CₒH₂ₒ mit o = 2 bis 12,
(CₚH₂ₚ-O)_{q}-CₚH₂ₚ mit p = 2 bis 4 und q = 1 bis 25, 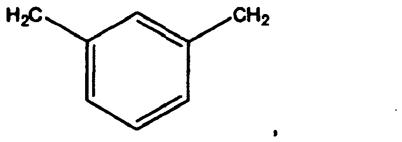 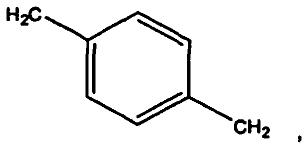 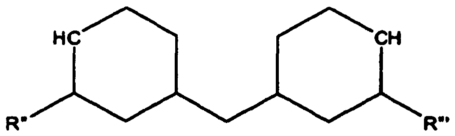 (R" und R"' stehen unabhängig voneinander für CH₃ oder H), oder 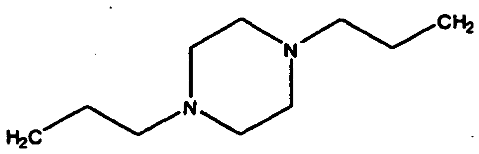 entspricht, oder Mischungen davon, zu Harnstoffurethanen umgesetzt werden, wobei zur Herstellung der Harnstoffurethane mindestens zwei strukturell verschiedene Monoisocyanat-Addukte, die sich im Rest R unterscheiden, mit den Diaminen umgesetzt werden, **dadurch gekennzeichnet, daß** die Umsetzung der mindestens zwei strukturell verschiedenen Monoisocyanat-Addukte mit den Diaminen in einer ionischen Flüssigkeit erfolgt, wobei ionische Flüssigkeiten organische Salze oder Gemische aus organischen Salzen sind, deren Schmelzpunkte unterhalb 80° C liegen.

2. Verfahren nach Anspruch 1, **dadurch gekennzeichnet, daß** zunächst mindestens zwei strukturell verschiedene Alkohole R-OH vermischt und anschließend mit einem 1,5 bis 5-fachen Überschuß von Toluylendiisocyanat umgesetzt werden, der nicht umgesetzte Teil des Toluylendiisocyanats aus dem Reaktionsgemisch entfernt wird, und die auf diese Weise erhaltenen Mischung aus strukturell verschiedenen Monoisocyanat-Addukten mit den Diaminen in einer ionischen Flüssigkeit zu Harnstoffurethanen umgesetzt wird.

3. Verfahren nach Anspruch 1, **dadurch gekennzeichnet, daß** zunächst mindestens zwei strukturell verschiedene Alkohole R-OH getrennt voneinander mit einem 1,5 bis 5-fachen Überschuß von Toluylendiisocyanat umgesetzt werden, und der nicht umgesetzte Teil des Toluylendilsocyanats aus den Reaktionsgemischen entfernt wird, und die auf diese Weise erhaltenen strukturell verschiedenen Monoisocyanat-Addukte miteinander vermischt und die erhaltene Mischung aus strukturell verschiedenen Monoisocyanat-Addukten mit den Diaminen in einem einer ionischen Flüssigkeit zu Harnstoffurethanen umgesetzt wird.

4. Verfahren nach einem oder mehreren der Ansprüche 2 oder 3, **dadurch gekennzeichnet, daß** der molare Anteil Monoisocyanat-Addukte in der Mischung aus strukturell verschiedenen Monoisocyanat-Addukten zwischen 20 und 80 % liegt, wobei die Summe der molaren Anteile der Monoisocyanat-Addukte 100 % beträgt.

5. Verfahren nach einem oder mehreren der Ansprüche 1 bis 4, **dadurch gekennzeichnet, daß** eine Lösung mit einem Feststoffgehalt von 5 bis 80 Gew.-% hergestellt wird.

6. Verfahren nach einem oder mehreren der Ansprüche 1 bis 5, **dadurch gekennzeichnet, daß** der molare Überschuß an Toluylendiisocyanat 2 bis 4 beträgt.

7. Verfahren nach einem oder mehreren der Ansprüche 1 bis 6, **dadurch gekennzeichnet, daß** ein Toluylendiisocyanat-Isomerengemisch mit einem Anteil von 50 bis 100 Gew.-% 2,4-Isomer verwendet wird.

8. Verfahren nach einem oder mehreren der Ansprüche 1 bis 7, wobei die ionische Flüssigkeit bei 25°C flüssig ist.

9. Verfahren nach einem oder mehreren der Ansprüche 1 bis 8, wobei die ionischen Flüssigkeiten die allgemeine Formel (I)
[A]ₙ⁺[Y]ⁿ⁻,
besitzen,
wobei n = 1 oder 2 ist und
das Anion [Y]ⁿ⁻ gewählt ist aus der Gruppe bestehend aus Tetrafluoroborat [BF₄]⁻, Tetrachloroborat [BCl₄]⁻, Phosphat [PO₄]³⁻, Alkylphosphat [ROPO₃]²⁻ / [ROR'OPO₂]⁻, Hexafluorophosphat [PF₆]⁻, Hexafluoroantimonat [SbF₆]⁻, Hexafluoroarsenat [AsF₆]⁻, Tetrachloroaluminat [AlCl₄]⁻, Trichlorozinkat [ZnCl₃]⁻. Dichlorocuprat [CuCl₂]⁻, Sulfat [SO₄]²⁻, Alkylsulfat [R'-SO₄]⁻, Carbonat [CO₃]²⁻, Fluorosulfonat, [R'-COO]', [R'-SO₃]⁻ oder [(R'-SO₂)₂N]⁻, und R, und R' unabhängig voneinander ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-Aryl-Rest ist, der dessen Wasserstoffatome ganz oder teilweise durch Halogenatome substituiert sein können und das Kation [A]⁺ gewählt ist aus der Gruppe bestehend aus 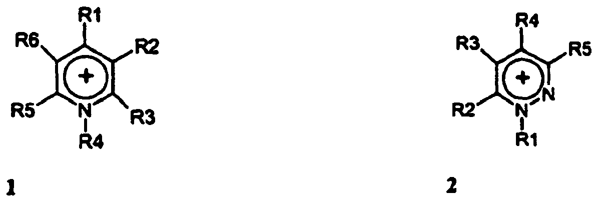 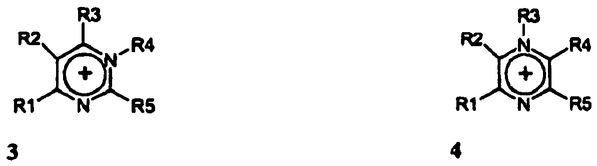 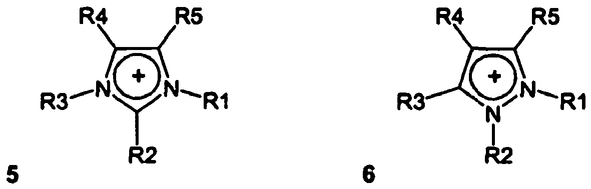 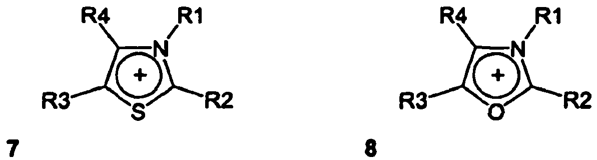 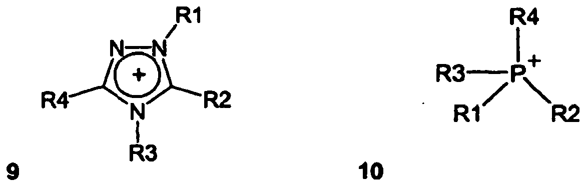 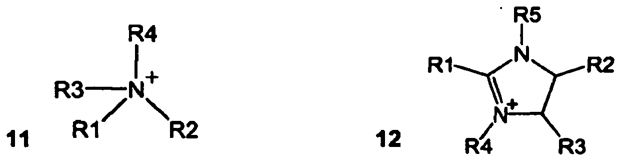 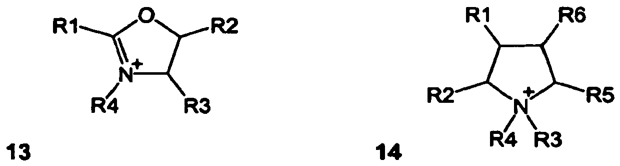 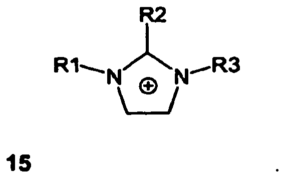
wobei die Reste R1, R2, R3, R4, R5 und R6 unabhängig voneinander gewählt sind aus der Gruppe bestehend aus (i) linearen oder verzweigten, gesättigten oder ungesättigten, aliphatischen oder alicyclischen Alkylgruppen mit 1 bis 20 Kohlenstoffatomen, die substituiert, beispielsweise mit einer Alkylgruppe mit 1-8 Kohlenstoffatomen und/oder Halogengruppe, oder unsubstituiert sein können; (ii) Heteroaryl-, Heteroaryl-C₁-C₆-Alkylgruppen mit 3 bis 8 Kohlenstoffatomen im Heteroarylrest und wenigstens einem Heteroatom ausgewählt aus N, O und S, der mit wenigstens einer Gruppe ausgewählt aus C₁-C₆-Alkylgruppen und/oder Halogenatomen substituiert sein können; (iii) Aryl-, Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenatom substituiert sein können; (iv) ein durch ein oder mehrere Heteroatome (N, O) unterbrochener linearer oder verzweigter aliphatischer und/oder cycloaliphatischer und/oder aromatischer Kohlenwasserstoffrest mit 2-40 Kohlenstoffatomen, der substituiert, beispielsweise mit einer Alkylgruppe mit 1-8 Kohlenstoffatomen und/oder Halogengruppe, oder unsubstituiert sein kann; (v) ein durch ein oder mehrere Funktionalitäten, ausgewählt aus der Gruppe -O-C(O)-, -(O)C-O-, -NH-C(O)-, -(O)C-NH-, -(CH₃)N-C(O)-, -(O)C-N(CH₃)-, -S(O)₂-O-, -O-S(O)₂-, -S(O)₂-NH-, -NH-S(O)₂-, -S(O)₂-N(CH₃)-, -N(CH₃)-S(O)₂-, unterbrochener linearer oder verzweigter aliphatischer Kohlenwasserstoffrest mit 2 - 20 Kohlenstoffatomen, der substituiert, beispielsweise mit einer Alkylgruppe mit 1-8 Kohlenstoffatomen und/oder Halogengruppe, oder unsubstituiert sein kann.

10. Verwendung der nach einem oder mehreren der Verfahren nach Ansprüchen 1 bis 9 hergestellten Lösungen zur Thixotropierung von Beschichtungsmitteln.

11. Lösungen der nach Anspruch 1 definierten Harnstoffurethane in ionischen Flüssigkeiten.

## Claims

1. A method for producing a solution comprising urea urethanes which is efficacious as a thixotropic agent, which method comprises reacting monohydroxy compounds of the general structure R-OH, where R is an n-alkyl radical or an i-alkyl radical of 4 to 22 carbon atoms, an alkenyl radical of 3 to 18 carbon atoms, a cycloalkyl radical or an aralkyl radical, a radical of the formula CₘH₂ₘ₊₁(O-CₙH₂ₙ)ₓ-, CₘH₂ₘ₊₁(OOC-CᵥH₂ᵥ)ₓ- or Z-C₆H₄(O-CₙH₂ₙ)ₓ- where m = 1-22, n = 2-4, x = 1-15 and v = 4 or 5 and Z is an alkyl radical of 1 to 12 carbon atoms, with a 1.5 to 5-fold excess of tolylene diisocyanate, removing the unconverted tolylene diisocyanate from the reaction mixture and reacting the monoisocyanate adducts thus obtained with diamines of the formula H₂N-R'-NH₂, where R' corresponds to
CₒH₂ₒ where o = 2 to 12,
(CₚH₂ₚ-O)_{q}-CₚH₂ₚ where p = 2 to 4 and q = 1 to 25, 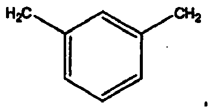 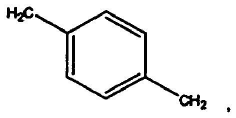 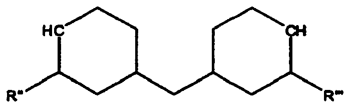 (R" and R"' are each independently CH₃ or H), or 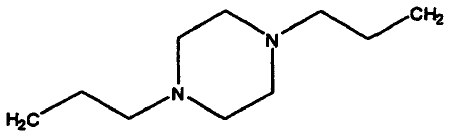 or mixtures thereof, to form urea urethanes, wherein the urea urethanes are produced by reacting at least two structurally different monoisocyanate adducts, which differ in the R radical, with the diamines, **characterized in that** the reaction of the at least two structurally different monoisocyanate adducts with the diamines takes place in an ionic liquid, whereas ionic liquids are organic salts or mixtures of organic salts having melting points below 80°C.

2. The method according to claim 1, **characterized in that** initially at least two structurally different alcohols R-OH are mixed and subsequently reacted with a 1.5 to 5-fold excess of tolylene diisocyanate, the unconverted tolylene diisocyanate is removed from the reaction mixture, and the mixture of structurally different monoisocyanate adducts which is obtained in this way is reacted with the diamines in an ionic liquid to form urea urethanes.

3. The method according to claim 1, **characterized in that** initially at least two structurally different alcohols R-OH are separately reacted with a 1.5 to 5-fold excess of tolylene diisocyanate, and the unconverted tolylene diisocyanate is removed from the reaction mixtures, and the structurally different monoisocyanate adducts obtained in this way are mixed with each other and the resulting mixture of structurally different monoisocyanate adducts is reacted with the diamines in an ionic liquid to form urea urethanes.

4. The method according to one or more of claims 2 or 3, **characterized in that** the molar fraction of monoisocyanate adducts in the mixture of structurally different monoisocyanate adducts is between 20 and 80%, wherein the sum total of the molar fractions of the monoisocyanate adducts is 100%.

5. The method according to one or more of claims 1 to 4, **characterized in that** a solution having a solids content of 5% to 80% by weight is produced.

6. The method according to one or more of claims 1 to 5, **characterized in that** the molar excess of tolylene diisocyanate is in the range from 2 to 4.

7. The method according to one or more of claims 1 to 6, **characterized in that** a tolylene diisocyanate isomer mixture having a 2,4-isomer fraction in the range from 50% to 100% by weight is used.

8. The method according to one or more of claims 1 to 7, wherein the ionic liquid is liquid at 25°C.

9. The method according to one or more of claims 1 to 8, wherein the ionic liquids have the general formula (I)
[A]ₙ⁺[Y]ⁿ⁻,
where n is = 1 or 2 and
the anion [Y]ⁿ⁻ is selected from the group consisting of tetrafluoroborate [BF₄]⁻, tetrachloroborate [BCl₄]⁻, phosphate [PO₄]³⁻, alkylphosphate [ROPO₃]²⁻ / [ROR'OPO₂]⁻, hexafluorophosphate [PF₆]⁻, hexafluoroantimonate [SbF₆]⁻, hexafluoroarsenate [AsF₆]⁻, tetrachloroaluminate [AlCl₄]⁻, trichlorozincate [ZnCl₃]⁻, dichlorocuprate [CuCl₂]⁻, sulfate [SO₄]²⁻, alkylsulfate [R'-SO₄]⁻, carbonate [CO₃]²⁻, fluorosulfonate, [R'-COO]⁻, [R'-SO₃]⁻ or [(R'-SO₂)₂N]⁻, and R, and R' is each independently a linear or branched aliphatic or alicyclic alkyl radical containing 1 to 12 carbon atoms, or a C₅-C₁₈-aryl, C₅-C₁₈-aryl-C₁-C₆-alkyl or C₁-C₆-alkyl-C₅-C₁₈-aryl radical, the hydrogen atoms of which can be wholly or partly replaced by halogen atoms, and the cation [A]⁺ is selected from the group consisting of 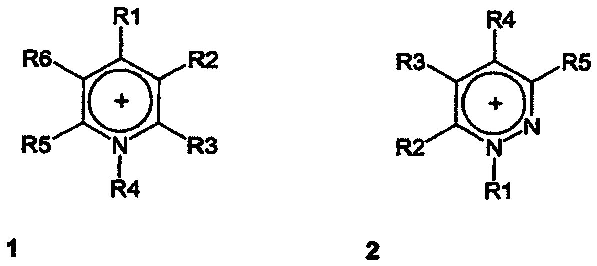 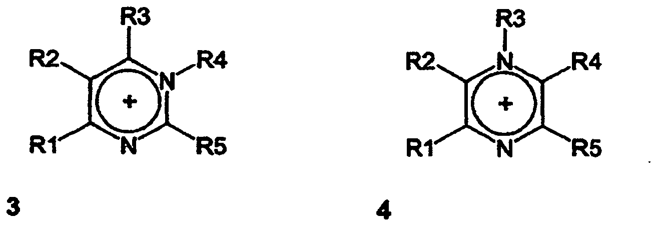 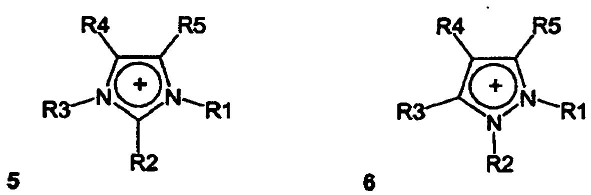 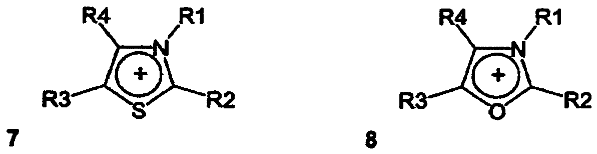 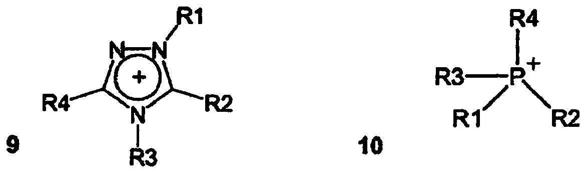 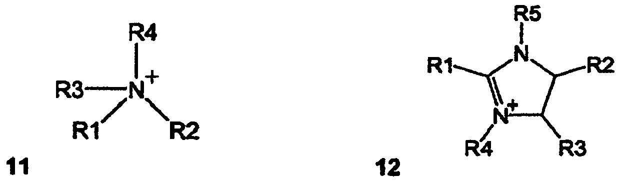 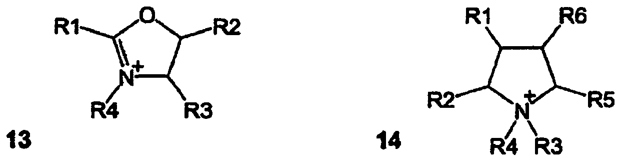 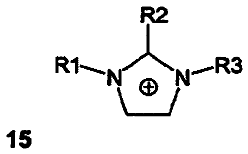
where the radicals R1, R2, R3, R4, R5 and R6 are each independently selected from the group consisting of (i) linear or branched, saturated or unsaturated, aliphatic or alicyclic alkyl groups of 1 to 20 carbon atoms, which can be substituted, for example with an alkyl group of 1-8 carbon atoms and/or halogen group, or unsubstituted; (ii) heteroaryl, heteroaryl-C₁-C₆-alkyl groups having 3 to 8 carbon atoms in the heteroaryl radical and at least one heteroatom selected from N, O and S, which radical can be substituted with at least one group selected from C₁-C₆-alkyl groups and/or halogen atoms; (iii) aryl, aryl-C₁-C₆-alkyl groups having 5 to 12 carbon atoms in the aryl radical, which may optionally be substituted with at least one C₁-C₆-alkyl group and/or a halogen atom; (iv) a linear or branched aliphatic and/or cycloaliphatic and/or aromatic hydrocarbon radical of 2-40 carbon atoms which is interrupted by one or more heteroatoms (N, O) and which can be substituted, for example with an alkyl group having 1-8 carbon atoms and/or halogen group, or unsubstituted; (v) a linear or branched aliphatic hydrocarbon radical of 2-20 carbon atoms which is interrupted by one or more functionalities selected from the group -O-C(O)-, -(O)C-O-, -NH-C(O)-, -(O)C-NH-, -(CH₃)N-C(O)-, -(O)C-N(CH₃)-, -S(O)₂-O-, -O-S(O)₂-, -S(O)₂-NH-, -NH-S(O)₂-, -S(O)₂-N(CH₃)-, -N(CH₃)-S(O)₂- and which can be substituted, for example with an alkyl group of 1-8 carbon atoms and/or halogen group, or unsubstituted.

10. The use of the solutions obtained according to one or more of the methods according to claims 1 to 9, for thixotroping coating materials.

11. A solution in an ionic liquid of the urea urethane defined according to claim 1.

## Revendications

1. Procédé de production d'une solution active en tant qu'agent thixotropique, contenant des uréthanes urées, dans lequel des composés monohydroxy de structure générale R-OH, où R repésente un groupement n-alkyle ou un groupement i-alkyle avec 4 à 22 atomes de carbone, un groupement alcényle avec 3 à 18 atomes de carbone, un groupement cycloalkyle ou un groupement aralkyle, un groupement de formule CₘH₂ₘ₊₁(O-CₙH₂ₙ)ₓ-, CₘH₂ₘ₊₁(OOC-CᵥH₂ᵥ)ₓ- ou Z-C₆H₄(O-CₙH₂ₙ)ₓ- avec m = 1 - 22, n = 2 - 4, x = 1 - 15 et v = 4 ou 5 et Z représente un groupement alkyle avec 1 à 12 atomes de carbone, sont réagis avec un excès de 1,5 à 5 fois de toluylène diisocyanate, la partie non réagie du toluylène diisocyanate est éliminée du mélange réactionnel et les adduits de monocyanate ainsi obtenus sont réagis avec des diamines de formule H₂N-R'-NH₂, dans laquelle R' correspond à
CₒH₂ₒ avec 0 = 2 à 12,
(CpH₂ₚ-O)_{q}-CₚH₂ₚ avec p = 2 à 4 et q = 1 à 25, 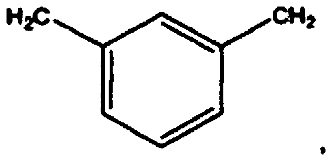 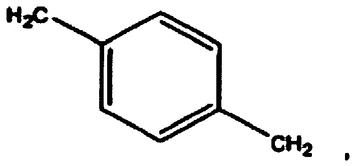 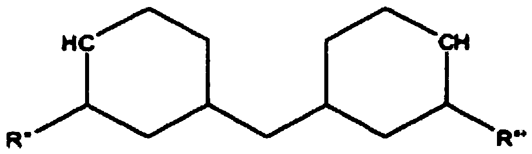 (R" et R"' représentent, indépendamment l'un de l'autre, CH₃ ou H), ou 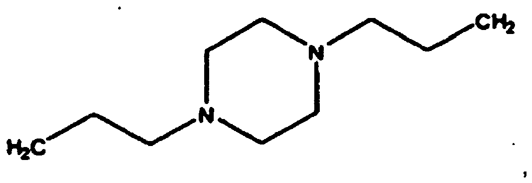 ou des mélanges de ceux-ci, pour obtenir des uréthanes urées, dans lequel au moins deux adduits de monoisocyanate structurellement différents dans le groupement R sont réagis avec les diamines pour la production des uréthanes urées, **caractérisé en ce que** la réaction des au moins deux adduits de monoisocyanate structurellement différents avec les diamines s'effectue dans un liquide ionique, les liquides ioniques étant des sels organiques ou mélanges des sels organiques ayant un point de fusion inférieur à 80 °C.

2. Procédé selon la revendication 1, **caractérisé en ce que** au moins deux alcools R-OH structurellement différents sont d'abord mélangés et puis réagis avec un excès de 1,5 à 5 fois de toluylène diisocyanate, la partie non réagie du toluylène diisocyanate est éliminée du mélange réactionnel, et le mélange ainsi obtenu des adduits de monoisocyanate structurellement différents est réagi avec les diamines dans une liquide ionique pour obtenir des uréthanes urées.

3. Procédé selon la revendication 1, **caractérisé en ce que** au moins deux alcools R-OH structurellement différents sont d'abord réagis, séparément l'un de l'autre, avec un excès de 1,5 à 5 fois de toluylène diisocyanate et la partie non réagie du toluylène diisocyanate est éliminée des mélanges réactionnels, les adduits de monoisocyanate structurellement différents ainsi obtenus sont mélangés l'un avec l'autre, et le mélange ainsi obtenu des adduits de monoisocyanate structurellement différents est réagi avec les diamines dans une liquide ionique pour obtenir des uréthanes urées.

4. Procédé selon l'une ou plusieurs des revendications 2 ou 3, **caractérisé en ce que** la proportion molaire des adduits de monoisocyanate dans le mélange des adduits de monoisocyanate structurellement différents est entre 20 et 80 %, la somme des proportions molaires des adduits de monoisocyanate étant de 100 %.

5. Procédé selon l'une ou plusieurs des revendications 1 à 4, **caractérisé en ce qu'**une solution avec une teneur en matières solides de 5 à 80 % en poids est préparée.

6. Procédé selon l'une ou plusieurs des revendications 1 à 5, **caractérisé en ce que** l'excès molaire du toluylène diisocyanate est de 2 à 4.

7. Procédé selon l'une ou plusieurs des revendications 1 à 6, **caractérisé en ce qu'**un mélange des isomères de toluylène diisocyanate avec une proportion de 50 à 100 % en poids de l'isomère 2,4 est utilisé.

8. Procédé selon l'une ou plusieurs des revendications 1 à 7, dans lequel le liquide ionique est dans l'état liquide à une temperature de 25 °C.

9. Procédé selon l'une ou plusieurs des revendications 1 à 8, dans lequel les liquides ioniques présentent la formule générale (I)
[A]ₙ⁺[Y]ⁿ⁻,
où n = 1 ou 2 et
l'anion [Y]ⁿ⁻ est choisi dans le groupe consistant en tétrafluoroborate [BF₄]⁻, tétrachloroborate [BCl₄]⁻, phosphate [PO₄]³⁻, alkylphosphate [ROPO₃]²⁻ / [ROR'OPO₂]⁻, hexafluorophosphate [PF₆]⁻, hexafluoroantimonate [SbF₆]⁻, hexafluoroarsenate [AsF₆]⁻, tétrachloroaluminate [AlCl₄]⁻, trichlorozincate [ZnCl₃]⁻, dichlorocuprate [CuCl₂]⁻, sulfate [SO₄]²⁻, alkylsulfate [R'-SO₄]⁻, carbonate [CO₃]²⁻, fluorosulfonate, [R'-COO]-, [R'-SO₃]⁻ ou [(R'-SO₂)₂N]⁻, et R et R' sont, indépendamment l'un de l'autre, un groupement alkyle linéaire ou branché, aliphatique ou alicyclique et contenant 1 à 12 atomes de carbone, ou un groupement C₅-C₁₈-aryle, C₅-C₁₈-aryle-C₁-C₆-alkyle- ou C₁-C₆-alkyle-C₅-C₁₈-aryle-, dont les atomes d'hydrogène peuvent être totalement ou partiellement substitués par des atomes d'halogène, et le cation [A]⁺ est choisi dans le groupe consistant en 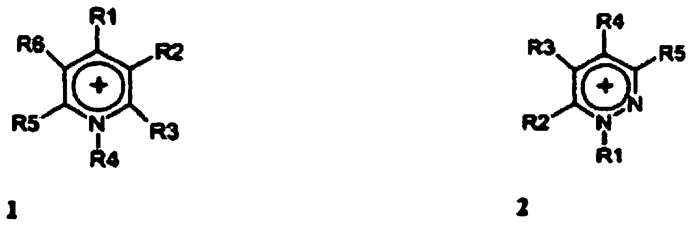 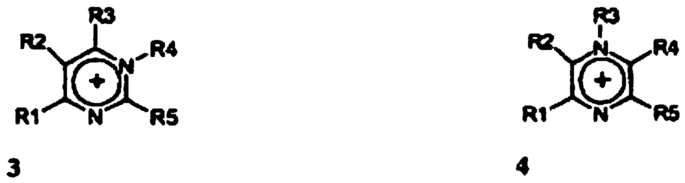 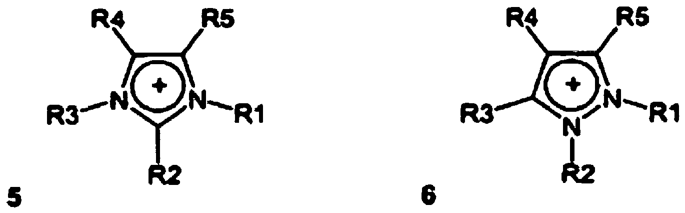 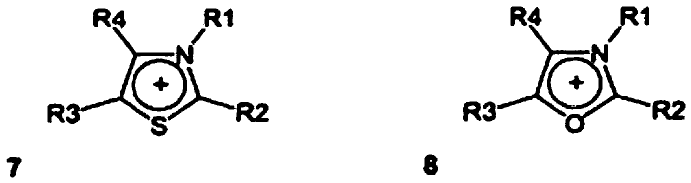 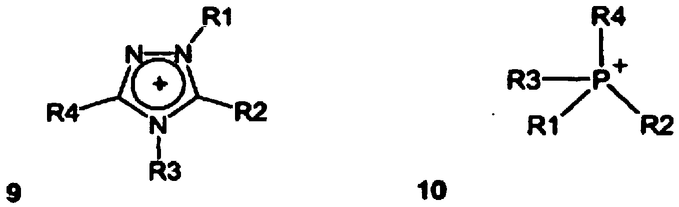 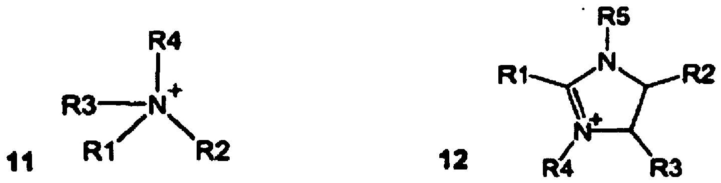 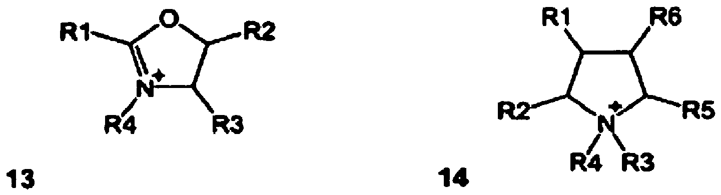 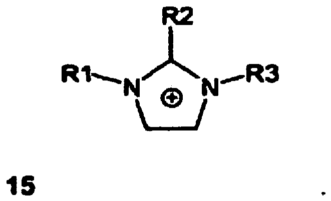
où les groupements R1, R2, R3, R4, R5 et R6 sont, indépendamment les uns des autres, choisi dans le groupe consistant en (i) des groupes alkyle linéaires ou ramifiés, saturés ou insaturés, aliphatiques ou alicycliques avec 1 à 20 atomes de carbone qui peuvent être substitués, par exemple avec un groupe alkyle avec 1 à 8 atomes de carbone et/ou un groupe halogène, ou non substitués ; (ii) des groupes hétéroaryle, hétéroaryle-C₁-C₆-alkyle avec 3 à 8 atomes de carbone dans le groupement hétéroaryle et au moins un hétéroatome choisi entre N, O et S, et qui peuvent être substitués avec un groupe choisi entre des groupes C₁-C₆-alkyle et/ou des atomes d'halogène ; (iii) des groupes aryle, aryle-C₁-C₆-alkyle avec 5 à 12 atomes de carbone dans le groupement aryle, qui peuvent être substitués, le cas échéant, avec au moins un groupe C₁-C₆-alkyle et/ou un atome d'halogène ; (iv) un groupement hydrocarbure aliphatique et/ou cycloaliphatique et/ou aromatique, linéaire ou ramifié et interrompu par un ou plusieurs hétéroatomes (N, O), avec 2 - 40 atomes de carbone, qui peut être substitué, par exemple avec un groupe alkyle avec 1 - 8 atomes de carbone et/ou un groupe halogène, ou non substitué ; (v) un groupement hydrocarbure aliphatique linéaire ou ramifié et interrompu par une ou plusieurs fonctionnalités, choisies dans le groupe consistant en -O-C(O)-, -(O)C-O-, - NH-C(O)-, -(O)C-NH-, -(CH₃)N-C(O)-, -(O)C-N(CH₃)-, -S(O)₂-O-, -O-ₛ(O)₂-, -S(O)₂-NH-, -NH-S(O)₂-, -S(O)₂-N(CH₃)-, -N(CH₃)-S(O)₂-, avec 2 - 20 atomes de carbone, qui peut être substitué, par exemple avec un groupe alkyle avec 1 - 8 atomes de carbone et/ou un groupe halogène, ou non substitué.

10. Utilisation de la solution préparée selon le procédé selon l'une ou plusieurs des revendications 1 à 9 pour la thixotropisation des agents de revêtement.

11. Solutions des uréthanes urées définies dans la revendication 1 dans des liquides ioniques.
